# EUROPEAN PATENT SPECIFICATION

(11) **EP 0 541 624 B1**
(45) Date of publication and mention of the grant of the patent: **12.06.1996**
(21) Application number: 91913795.0
(22) Date of filing: 29.07.1991
(51) Int. Cl.: A61M 5/32

(54) **DEVICE FOR REMOVAL AND CONTAINMENT OF USED NEEDLES**
VORRICHTUNG ZUM LÖSEN UND AUFBEWAHREN GEBRAUCHTER KANÜLEN
DISPOSITIF DE SEPARATION ET DE RECUPERATION D'AIGUILLES UTILISEES

(30) Priority: 31.07.1990 GB 9016751
(43) Date of publication of application: 19.05.1993
(73) Proprietor: SAMS, Bernard, London N11 3EU (GB)
(72) Inventor: SAMS, Bernard, London N11 3EU (GB)
(74) Representative: Gillam, Francis Cyril (GB)
(86) International application number: GB9101277
(87) International publication number: WO9202265

(56) References cited:
- WO-A-84/02674
- FR-A- 2 613 230
- GB-A- 1 396 464
- US-A- 3 796 359
- US-A- 4 380 292

## Description

The present invention relates to a device for breaking a needle away from an article on which the needle is mounted, such as a hypodermic syringe, for the safe disposal of the needle.

### BACKGROUND TO THE INVENTION

Fine bore or capillary needles are often used as the operative end of a device to penetrate the skin of a person to inject or remove blood or other bodily fluids, for example lumbar fluids. When such needles have been used, they are usually contaminated with the blood or fluid of the person and for health reasons must be cleaned or sterilised before re-use. This is an increasingly expensive and time consuming operation and such needles and hypodermic syringes incorporating them have been put up in disposable form so that they are discarded after a single use. However, the disposal has to be in such a manner as to prevent unauthorised re-use of the syringe and such as to minimise the risk of someone contacting the discarded needle and becoming infected therefrom.

To this end, it is current practice to cut the needles off hypodermic syringes after use so that the body of the syringe cannot be re-used and the needles can be disposed of separately. Typically, a hypodermic syringe needle is made from stainless steel capillary tubing which is both flexible and hard to cut. Therefore, present cutting tools require hardened or tool steel blades which are expensive. Furthermore, cutting the needle off with such cutters often leaves a sharp projecting stump which can cause as much damage as the end of a conventional needle and may still carry contaminants which could infect a cut made by the stump. Having cut the needle off the syringe, the user is still presented with the problem of disposal of the needle, which is now aggravated since the user has to grip the needle itself and cannot use the body of the syringe to manoeuvre the needle.

It has been proposed in UK Patent Application No. 2214082A to provide the syringe with a hollow needle cover which can be flexed about the axis of the needle to snap the needle mounting off the syringe. However, if the needle cover is misplaced during use of the syringe, as may well occur, no other means are provided for rendering the syringe safe after use. Furthermore, since the cover is intended to be readily removed from the needle prior to use, it may not retain the needle firmly once the needle has been broken away from the syringe body. As a result, the needle may escape from the cover prior to disposal and present a double ended hazard to the user.

I have now devised an improved method and device for breaking a needle away from a support, notably from a hypodermic syringe, which reduces the above problems.

### SUMMARY OF THE INVENTION

Accordingly, the present invention provides a needle disposal device arranged to facilitate the breaking of a needle away from an article on which the needle is mounted for the subsequent safe storage of the broken needle, which device comprises a hollow body having an open face, there being a mass of material located within the body which material is axially penetrable by a needle but is resistant to lateral movement of a needle when inserted therein, the mass of material having a surface exposed through the face of the hollow body which surface may act as a fulcrum for rocking movement of said article when the needle thereof has fully been inserted in the material, which rocking movement causes flexing of the needle in the region of the junction between the needle and said article so as to break the needle away from said article and to be retained within the mass of material.

The invention also provides a method for separating a capillary bore needle from a supporting article at substantially the point at which the needle projects from the article, which method comprises inserting the needle essentially completely into the mass of material within the hollow body of a device of the invention and rocking the article transversely to the longitudinal axis of the needle whereby the needle is broken away from the article and is retained in the mass of material.

The invention can be applied to a wide range of forms of needle, for example fine drawn glass irrigation canulae, lumbar puncture needles and other metal fine bore needles attached to syringes or tubing for use in drawing blood samples. However, the invention is of especial application in separating stainless steel capillary bore needles from the bodies of disposable injection syringes. In such devices the needle is a sharp ended drawn stainless steel tube with an internal diameter of from 0.1 to 1.0 mm. One end of the needle is embedded in the body of the syringe during moulding of the plastic body of the syringe or is otherwise substantially rigidly secured to the body of the syringe, for example by means of a push fit mounting. Typically, the needle projects from 1 to 7 cms from the free end of the syringe. For convenience, the invention will be described hereinafter in terms of breaking such capillary needles from a one time use plastic disposable syringe.

The needle to be broken off is inserted substantially completely into the mass of material within the hollow body through an open face of the body. The hollow body can be defined by a recess in an end of a cap used to protect the needle prior to its use, an axial bore in the body of the plunger used to operate the syringe, or the body of a container such as a tub or tube. The hollow body can thus be a simple plastic open-topped tub or the like and many different shapes and form of container may be used.

The hollow body must have a depth sufficient to receive the full length of the needle to be broken from the syringe body, typically 1.5 to 7.5 cms. The body should have an internal capacity sufficient to hold a plurality of needles so that a single device can be used a number of times before it is necessary to dispose of the device and its load of needles. Typically, the device will be capable of holding from 10 to 100 needles.

Preferably, the hollow body has a removable top whereby the material within the hollow body can be exposed for use in breaking off a needle; and the lid then re-applied to close the body and retain the needle securely within the body. The lid also protects a user from accidental contact with the needles within the body.

A particularly preferred form for the mass of material into which the needle is inserted is a foamed plastic or the like material. I have found that this acts to restrain the shaft of the needle within the hollow body adequately to cause breakage of the needle when the syringe is swung about the axis of the needle.

I believe that the use of such a foamed material to provide the lateral restraint is novel and has the further advantage that the needle will be retained within the material, when broken away from the article.

The mass of material within the hollow body can take a wide range of forms. It must permit a needle easily to be inserted fully, and yet be sufficiently firm to prevent excessive lateral deflection of the needle during the flexing of the needle to break it. Typically, the material is one which will prevent the needle from deflecting by more than 30° to either side of the longitudinal axis of the needle when first inserted into the medium. It is particularly preferred that the medium permit the needle to deflect by less than 10° to either side of the needle axis. The degree of deflection may also depend upon the position of the needle relative to the side wall of the container, since that wall will also serve to restrain the deflection of the needle. The optimal restraining material for use with a given needle and container can readily be determined by simple trial and error tests to determine whether the needle snaps within the three to five flexings.

Thus, suitable materials include closed or open pored foamed plastics having a sufficient degree of resilience, rolled paper or fabric, bundles of smaller bore tubes held within a larger diameter hollow body, blocks of natural or synthetic rubber of other resins or polymers, and blocks or pads of randomly orientated fibres, such as felted materials. Such materials are typical of those into which a needle can be driven axially and which will restrain the needle shank against excessive lateral deflection when the syringe body is rocked laterally about the axis of the needle. A particularly preferred restraining material for present use is a closed pore polyurethane or other synthetic polymer having a compressibility ratio, ie. the percentage reduction in volume of a 1 cm cube of the material when subjected to a load of 1kg per square cm, of from 5 to 25%.

If desired, the mass of material may be formed from a series of cells of the material separated by rigid axial walls within the hollow body, so as increase the effective compressibility ratio due to the presence of the adjacent wall. If desired, the material can contain or be impregnated with a germicide or other treatment to reduce the risk of contamination to the user from a needle.

The device of the invention preferably also has means for gripping a needle and restricting withdrawal of the needle from the mass of material during flexing of the needle. This means can be provided by the upper surface of the mass of material where this grips the shank of the needle sufficiently. Alternatively, a separate gripping member can be provided, for example a rubber or soft plastic membrane or collar which is penetrated by the needle on entering the hollow body. The gripping member can be formed with pre-formed apertures through which the needle is to pass and such apertures can incorporate saw tooth lips or other means whereby retraction of the needle from the aperture is prevented.

The gripping member is located above, at or closely adjacent the exposed upper surface of the mass of material so that the shank of the needle is gripped closely adjacent the point at which it is mounted in the body of the syringe. As indicated above, the gripping member is conveniently provided by a membrane or sheet over the open face of the hollow body. This membrane or sheet is axially depressed as the end of the syringe carrying the needle bears against it when the needle has been inserted into the material. This serves to maintain the membrane in contact with the end of the body of the syringe and thus to ensure that flexing of the needle is maximised at this point so that breakage occurs there and not elsewhere along the length of the needle.

In use, the distal end of the needle of a used syringe is inserted into the hollow body through the open face thereof, and so into the mass of material within the body. The syringe body is pressed home against the exposed face of the mass of material or the membrane extending thereover. The body of the syringe is then rocked about the longitudinal axis of the needle, so flexing the needle about its junction with the syringe. Preferably, the syringe is rocked through the maximum angle possible in one plane and is not rotated about the axis of the needle, so as to maximise the flexing of the needle. It is therefore preferred that the upper surface of the device of the invention be substantially flat and present the minimum obstruction to the rocking movement of the syringe. Typically, the needle will snap off at its mounting in the body of the syringe after two or three flexes through 120 to 180° about the axis of the needle. When the needle has been snapped off, the syringe can be disposed of with reduced risk of the needle remains held within the material in the hollow body. The body can then be closed by re-applying its lid, to retain the broken needle securely.

### DESCRIPTION OF THE DRAWINGS

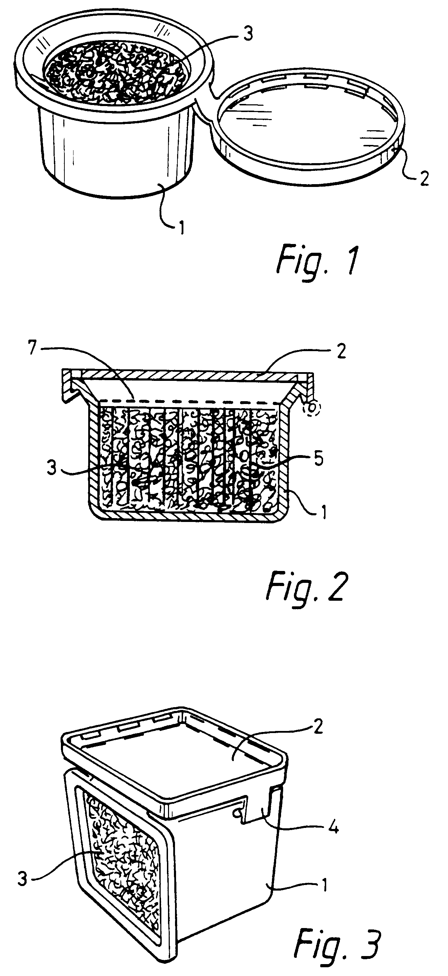

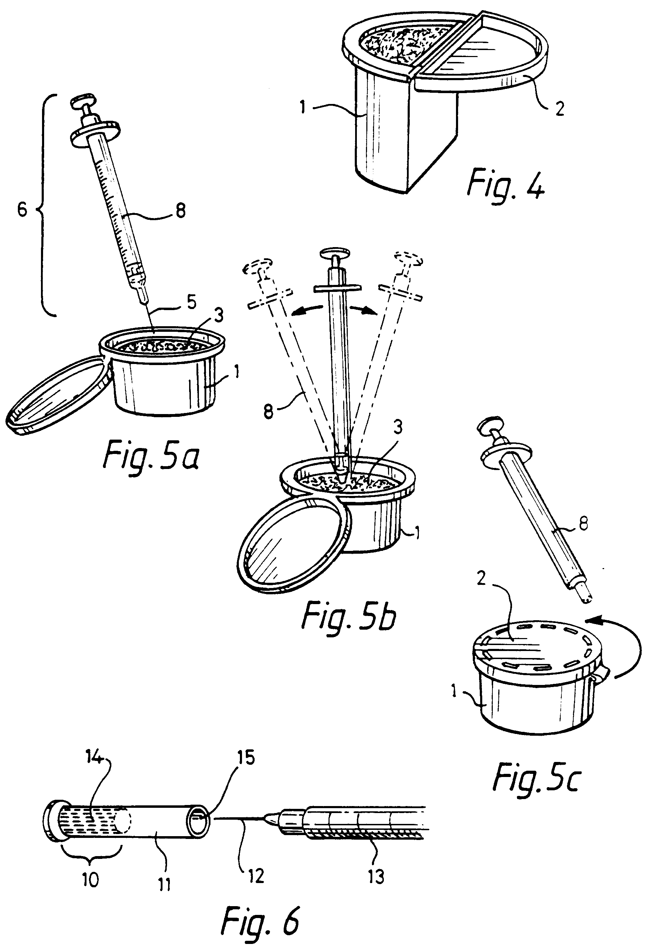

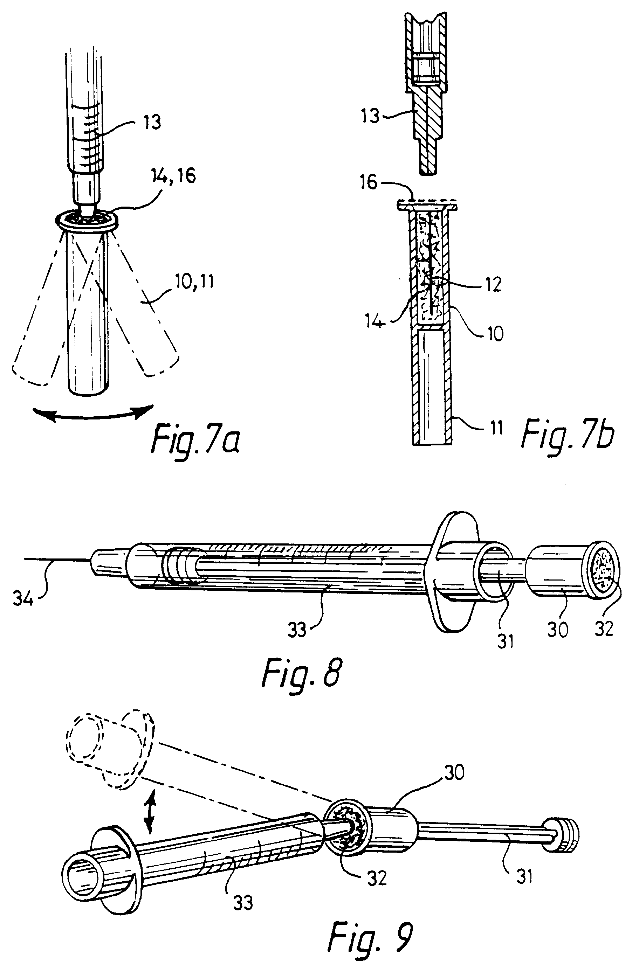

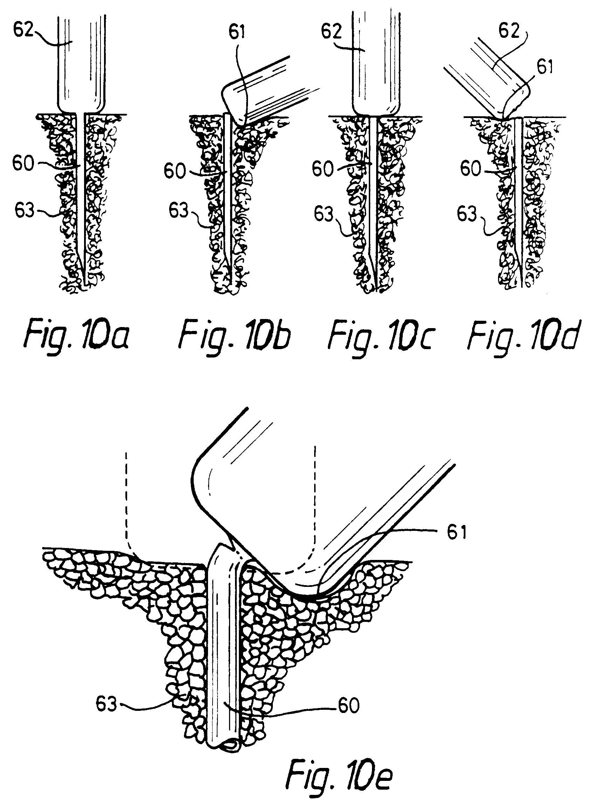

The invention will now be described by way of illustration only with respect to a number of preferred forms thereof as shown in the accompanying drawings, in which Figure 1 is a perspective view of a container containing a foamed plastic pad with a closable lid; Figure 2 is a vertical section through the container of Figure 1 with the lid in the closed position; Figures 3 and 4 are perspective views of alternative forms of the container of Figure 1; Figure 5a to 5c show the container of Figure 1 in use; Figure 6 shows an alternative form of the device of the invention and Figures 7a and 7b show that device in use; Figure 8 shows an alternative form of the device of Figure 6 and Figure 9 shows the device of Figure 8 in use; and Figure 10 illustrates the operation of the foamed plastic restraining member.

### DESCRIPTION OF THE PREFERRED EMBODIMENTS

The device of Figure 1 comprises a conventional cylindrical plastic tub 1 having an integral hinged top 2 and containing a pad 3 of closed pore polyester or Neoprene foam having a density of 200 Kgs per metre cube. The depth of the tub 1 and of page 3 are such that the needle 5 of a hypodermic syringe 6 can be fully inserted into the foam pad. In place of the foamed plastic, the pad 3 can be formed by winding cloth, papers, blotting paper or other fibrous material about a central core to provide a generally cylindrical pad with the interfaces between successive layers of the paper aligned axially so that a needle can be inserted between them.

The tub 1 can be of circular cross-section as shown in Figure 1 or can have a squared or semi-circular cross-section as shown in Figures 2 and 3 or any other suitable shape. As shown in Figure 2, the lid can be provided with clips 4 or other means whereby the lid is held in the open position during use.

The needle 5 of a syringe is pushed into the pad 3 until the free end of the syringe beds against the top face of the pad 3. The needle is held firmly within the pad both axially and laterally. If desired, a rubber or other membrane 7, shown dotted in Figure 2, can be applied over the top of the pad to enhance the axial grip on the needle 5. The body 8 of the syringe 6 is then flexed about the axis of the needle 5, which is prevented from following the flexing by the restraining action of pad 3 and the membrane 7. The needle will snap off at the point where it enters the syringe body to provide, as shown in Figure 5c, a syringe body without any significant projecting needle stump, the broken off section of the needle being retained within the pad 3, as shown in Figure 2, for subsequent disposal when the pad is full.

In the form of device shown in Figure 6, the container is provided by a cylindrical extension 10 to the protective cap 11 normally used to protect the needle 12 of the syringe 13 before use. A pad 14 of foamed plastic is mounted within the bore 15 of the extension 10 and the free, open end of the bore is closed with a transverse rubber or similar membrane 16, as shown dotted in Figure 7. In use, as shown in Figure 7a, the needle 12 is pushed through the membrane 16 and into the pad 14 until the free end of the syringe 13 beds against the outer face of the membrane 16 or pad 14. The syringe is then flexed to and fro until the needle snaps off and is retained in the bore 15 by means of the pad 14 and the membrane 16, as shown in Figure 7b.

In the device shown in figures 8 and 9, the push bottom end 30 of the plunger 31 of the syringe is formed as a hollow socket which carries a foam plastic pad 32 therein, optionally with a membrane over the open end of the pad 32. When the syringe has been used, the plunger 30 is pulled out of the barrel 33 of the syringe, the needle 34 is inserted into the pad 31 and broken off as shown in Figure 9 by flexing the syringe body to and fro.

As shown in Figure 10, the mass of foamed plastics material acts to provide at least part of the fulcrum effect required to localise the flexing of the needle by being trapped between the shank of the needle 60 and the shoulder 61 of the syringe body or the needle mounting 62 as shown in Figure 10e. This enables the needle to flex about a specific point as shown in Figures 10a to 10d, the remainder of the material 63 providing lateral restraint to the length of the shank of the needle.

## Claims

1. A needle disposal device arranged to facilitate the breaking of a needle away from an article on which the needle is mounted for the subsequent safe storage of the broken needle, which device comprises a hollow body (1,11,30) having an open face, there being a mass of material (3,14,32,63) located within the body which material is axially penetrable by a needle but is resistant to lateral movement of a needle when inserted therein, the mass of material having a surface exposed through the open face of the hollow body which surface may act as a fulcrum for rocking movement of said article when the needle thereof has fully been inserted in the material, which rocking movement causes flexing of the needle in the region of the junction between the needle and said article so as to break the needle away from said article and to be retained within the mass of material.

2. A device as claimed in Claim 1, characterised in that the mass of material comprises a foamed plastics.

3. A device as claimed in Claim 2, characterised in that the foamed plastics material undergoes a reduction of volume of from 5 to 25% when a 1 cm cube of the material is subjected to a load of 1kg per square cm.

4. A device as claimed in any of Claims 1 to 3, characterised in that the mass of material restricts lateral movement of an inserted needle to not more than 30° to either side of the longitudinal axis of the needle upon the flexing thereof by rocking said article.

5. A device as claimed in Claim 4, characterised in that the mass of material restricts said lateral movement to not more than 10°.

6. A device as claimed in any of the preceding Claims, characterised in that a flexible membrane (7,16) is provided across the opening to the hollow body.

7. A method for separating a capillary bore needle from a supporting article at substantially the point at which the needle projects from the article, which method is characterised in that it comprises inserting the needle essentially completely into the mass of material within the hollow body of a device as claimed in any one of claims 1 to 5 and rocking the article transversely to the longitudinal axis of the needle whereby the needle is broken away from the article and is retained in the mass of material.

8. A method as claimed in Claim 6, characterised in that said article is a disposable hypodermic syringe.

9. A method as claimed in Claim 7 or Claim 8, characterised in that the compression of the mass of material as the needle is flexed provides the fulcrum about which the needle bends to cause breaking of the needle.

## Patentansprüche

1. Nadelentsorgungsvorrichtung, die dazu eingerichtet ist, das Wegbrechen einer Nadel von einem Gegenstand, auf dem die Nadel montiert ist, für die nachfolgende sichere Aufbewahrung der abgebrochenen Nadel zu erleichtern, welche Vorrichtung einen hohlen Körper (1, 11, 30) mit einer offenen Seite aufweist, wobei eine innerhalb des Körpers befindliche Masse an Material (3, 14, 32, 63) vorgesehen ist, welches Material von einer Nadel axial durchdringbar ist, aber einer seitlichen Bewegung einer darin eingesetzten Nadel widersteht, wobei die Masse an Material eine durch die offene Seite des hohlen Körpers freiliegende Oberfläche hat, welche Oberfläche als eine Hebelstütze für eine Hin- und Herbewegung des Gegenstandes dienen kann, wenn dessen Nadel vollständig in das Material eingesetzt worden ist, wobei die Hin- und Herbewegung ein Biegen der Nadel in dem Bereich der Verbindung zwischen der Nadel und dem Gegenstand bewirkt, um so die Nadel von dem Gegenstand wegzubrechen und innerhalb der Masse an Material zurückgehalten zu werden.

2. Vorrichtung nach Anspruch 1, dadurch gekennzeichnet, daß die Masse an Material einen geschäumten Kunststoff aufweist.

3. Vorrichtung nach Anspruch 2, dadurch gekennzeichnet, daß das geschäumte Kunststoffmaterial eine Volumenverminderung von 5% bis 25% erfährt, wenn ein 1cm-Würfel des Materials einer Belastung von 1 kg pro cm² ausgesetzt wird.

4. Vorrichtung nach einem der Ansprüche 1 bis 3, dadurch gekennzeichnet, daß die Masse an Material die seitliche Bewegung einer eingesetzten Nadel bei deren Biegen durch Hin- und Herbewegen des Gegenstandes auf nicht mehr als 30° zu jeder Seite des Längsachse der Nadel einschränkt.

5. Vorrichtung nach Anspruch 4, dadurch gekennzeichnet, daß die Masse an Material die seitliche Bewegung auf nicht mehr als 10° einschränkt.

6. Vorrichtung nach einem der vorhergehenden Ansprüche, dadurch gekennzeichnet, daß über der Öffnung zu dem hohlen Körper eine flexible Membran (7, 16) vorgesehen ist.

7. Verfahren zum Trennen einer mit einer kapillaren Bohrung versehenen Nadel von einem haltenden Gegenstand an im wesentlichen der Stelle, an der die Nadel von dem Gegenstand vorspringt, wobei das Verfahren dadurch gekennzeichnet ist, daß es ein im wesentlichen vollständiges Einsetzen der Nadel in die Masse an Material innerhalb des hohlen Körpers einer Vorrichtung nach einem der Ansprüche 1 bis 5 und ein Hin- und Herbewegen des Gegenstands quer zur Längsachse der Nadel aufweist, wodurch die Nadel von dem Gegenstand weggebrochen wird und in der Masse an Material zurückgehalten wird.

8. Verfahren nach Anspruch 6, dadurch gekennzeichnet, daß der Gegenstand eine Einwegspritze ist.

9. Verfahren nach Anspruch 7 oder 8, dadurch gekennzeichnet, daß die Zusammendrückung der Masse an Material, wenn die Nadel gebogen wird, die Hebelunterlage bereitstellt, um die sich die Nadel biegt, um ein Brechen der Nadel zu bewirken.

## Revendications

1. Dispositif d'élimination d'aiguille, agencé pour faciliter la rupture d'une aiguille pour la détacher d'un objet sur lequel l'aiguille est montée, pour le stockage ultérieur et sans riques de l'aiguille rompue, lequel dispositif comprend un corps creux (1, 11, 30) ayant une face ouverte, une masse de matériau étant placée à l'intérieur du corps, lequel matériau est pénétrable axialement par une aiguille mais résistant au mouvement latéral d'une aiguille une fois qu'elle y est insérée, la masse de matériau ayant une surface laissée à découvert par la face ouverte du corps creux, laquelle surface peut se comporter comme un pivot pour le mouvement de basculement dudit objet une fois son aiguille complètement insérée dans le matériau, lequel mouvement de basculement provoque la flexion de l'aiguille dans la région de la jonction entre l'aiguille et ledit objet de façon que l'aiguille se détache dudit objet par rupture et reste retenue à l'intérieur de la masse de matériau.

2. Dispositif selon la revendication 1, caractérisé en ce que la masse de matériau comprend un plastique alvéolaire.

3. Dispositif selon la revendication 2, caractérisé en ce que le matériau plastique alvéolaire subit une réduction de volume de 5 à 25% quand 1cm cube du matériau est soumis à une charge de 1kg par cm carré.

4. Dispositif selon l'une quelconque des revendications 1 à 3, caractérisé en ce que la masse de matériau restreint le mouvement latéral d'une aiguille qui est insérée, à 30° au maximum de part et d'autre de l'axe longitudinal de l'aiguille lors de sa flexion par basculement dudit objet.

5. Dispositif selon la revendication 1, caractérisé en ce que la masse de matériau restreint ledit mouvement latéral à 10° au maximum.

6. Dispositif selon l'une quelconque des revendications précédentes, caractérisé en ce qu'une membrane flexible (7, 16) est prévue en travers de l'ouverture du corps creux.

7. Procédé pour séparer une aiguille à trou capillaire d'un objet qui la supporte, sensiblement à l'endroit où l'aiguille dépasse de l'objet, procédé caractérisé en ce qu'il comprend l'insertion sensiblement complète de l'aiguille dans la masse de matériau contenue dans le corps creux d'un dispositif selon l'une quelconque des revendications 1 à 5, et le basculement de l'objet transversalement par rapport à l'axe longitudinal de l'aiguille, de sorte que l'aiguille se détache de l'objet par rupture et se trouve retenue dans la masse de matériau.

8. Procédé selon la revendication 7, caractérisé en ce que ledit objet est une seringue hypodermique à usage unique.

9. Procédé selon la revendication 7 ou 8, caractérisé en ce que la compression de la masse de matériau lors de la flexion de l'aiguille fournit le pivot autour duquel l'aiguille fléchit jusqu'à provoquer la rupture de l'aiguille.
